# EUROPEAN PATENT APPLICATION

(11) **EP 4 772 620 A1**
(43) Date of publication of application: **08.07.2026**
(21) Application number: 25806891.5
(22) Date of filing: 14.05.2025
(51) Int. Cl.: C12N 9/18, C12P 7/64, C11C 1/04, C11C 3/06

(54) **PREPARATION METHOD FOR AND USE OF MEDIUM-CHAIN TRIGLYCERIDE**

(30) Priority: 22.05.2024 CN 202410641079
(71) Applicant: Guangzhou Cocolipids Industrial Co., Ltd., Guangzhou, Guangdong 510535 (CN); Guangdong Sihai Biotechnology Co., Ltd., Guangzhou, Guangdong 513042 (CN)
(72) Inventor: AI, Hongzeng, Guangzhou, Guangdong 510535 (CN); ZHENG, Yilan, Guangzhou, Guangdong 510535 (CN); YING YE, Alfredo, Guangzhou, Guangdong 510535 (CN); SHAO, Xin, Guangzhou, Guangdong 510535 (CN); LI, Xiang, Guangzhou, Guangdong 510535 (CN); LIAO, Yuhuan, Guangzhou, Guangdong 510535 (CN); SUN, Qimeng, Guangzhou, Guangdong 510535 (CN); FENG, Qiang, Guangzhou, Guangdong 510535 (CN); XIE, Xiaodong, Guangzhou, Guangdong 510535 (CN); CHEN, Tao, Guangzhou, Guangdong 510535 (CN)
(74) Representative: Petraz, Gilberto Luigi
(86) International application number: PCT/CN2025/094733
(87) International publication number: WO 2025/241952

(57) **Abstract**

The invention belongs to the technical field of light industrial oil, and particularly discloses a preparation method for and a use of a medium chain triglyceride. In the invention, a phospholipase A₂ is specifically chemically-modified by using a modification solution prepared from EDC or a mixture of EDC and iodoacetamide to obtain a modified phospholipase A₂ first, the modified phospholipase A₂ is used as a catalyst for catalyzing hydrolysis of coconut oil, and C8 and C10 fatty acids are enriched by specific hydrolysis, and then esterified with glycerol to synthesize the medium chain triglyceride. In the invention, the specifically chemically-modified phospholipase A₂ shows improved hydrolysis specificity for the C8 and C10 fatty acids in the coconut oil, so that a light phase component having a high content of C8 and C10 fatty acids is obtained after molecular distillation of a hydrolysate, and by means of subsequent esterification with the glycerol, the medium chain triglyceride in which a content of C8 and C10 reaches 95% can be obtained, and used in the fields of food, nutrition and medicine.

## Description

### TECHNICAL FIELD

The present invention belongs to the technical field of light industrial oil, and particularly relates to a preparation method for and a use of a medium chain triglyceride.

### BACKGROUND

Medium chain fatty acids (MCFAs) usually refer to fatty acids having six to twelve carbon atoms, and mainly include caprylic acid (chemical abbreviation: C8, containing 8 carbon atoms) and capric acid (C10, with 10 carbon atoms), along with small amounts of caproic acid (C6, with 6 carbon atoms) and lauric acid (C12, with 12 carbon atoms), which are esterified with three hydroxyl groups of glycerol to form medium chain triglycerides (MCTs), wherein the most typical medium chain triglyceride refers to a complex of saturated caprylic triglyceride, saturated capric triglyceride and saturated caprylic-capric triglyceride.

The medium chain triglycerides are digested and broken down approximately ten times faster than long chain triglycerides, and this is because the MCFAs generated after hydrolysis by pancreatic lipase in the body are directly transported to the liver via the portal vein and then rapidly broken down in the liver to generate energy quickly. Therefore, based on the unique metabolic properties, the medium chain triglycerides have been widely used in the fields of food, nutrition, medicine, etc. The medium chain triglycerides are used in clinical nutrition for treating malnutrition, fat malabsorption, celiac disease, chronic pancreatic insufficiency, intestinal fistula, bile duct obstruction, hyperlipidemia, high cholesterol, obstructive jaundice, inflammatory bowel disease, fatty liver disease, pancreatitis, and other related diseases. In addition, the medium chain triglycerides are widely used in many pharmaceutical formulations due to the favorable properties, such as antioxidant activity, good solubility, low viscosity and low melting point, as well as the ability to reduce bacterial heat resistance. The medium chain triglycerides may also enhance the human body's digestion and absorption of fat-soluble vitamin E, thereby serving as dietary supplements for patients with dyspepsia or impaired energy absorption. When drugs are prepared as emulsified formulations by using the medium chain triglycerides, the bioavailabilities of the drugs are all significantly improved.

There are limited natural sources of medium chain triglycerides, and a primary industrial production method involves chemical synthesis, in which a non-enzymatic catalyst is employed to catalyze the esterification of medium chain fatty acids with glycerol to produce medium chain triglycerides. Although the chemical method for preparing the medium chain triglycerides has a simple technological process, a low energy consumption and a short reaction time, there are also a large number of by-products, and the by-products are difficult to separate, thereby easily leading to product contamination, unpleasant odors, and other undesirable properties.

### SUMMARY

In view of the above problems in the prior art, the inventor provides a preparation method for and a use of a medium chain triglyceride. In the method, a chemically modified phospholipase A₂ is used to catalyze hydrolysis of coconut oil, and C8 and C10 fatty acids in the coconut oil are selectively hydrolyzed to achieve the purpose of enriching raw materials for synthesizing the medium chain triglyceride, so that green catalytic conversion is implemented, reaction conditions are mild and have little influence on product properties, and production costs are further reduced. Therefore, the medium chain triglyceride can be used in the fields of food, nutrition and medicine.

A first objective of the present application is to provide a method for improving a catalytic activity of a specifically chemically-modified phospholipase A₂, wherein the method includes the following steps: mixing a modification solution with a phospholipase A₂ for reaction, removing an upper-layer solvent by centrifugation after the reaction, centrifugally ultrafiltering a remaining solution to obtain a concentrated solution, and diluting the concentrated solution to obtain the specifically chemically-modified phospholipase A₂, wherein the modification solution is prepared from 1-(3-dimethylaminopropyl)-3-ethylcarbodiimide hydrochloride (EDC) or a mixture of EDC and iodoacetamide.

Chemical modification refers to forming or destroying covalent parts or partial covalent bonds of an enzyme molecule by introducing or removing chemical groups. In the present application, the iodoacetamide reacts with a sulfhydryl group on cysteine and the EDC serves as an activating reagent for a carboxyl group, and by means of combinatorial modification of a side chain group of an enzyme amino acid, an enzyme protein is molecularly modified by "cleaving" and "splicing" a main chain of the enzyme molecule and modifying the side chain group, which leads to changes in certain properties and functions of an enzyme, and alters catalysis characteristics, so that products meeting specific requirements are prepared.

Preferably, the modification solution is a modification solution with a concentration of 0.1-0.5 mol/L, which is prepared by dissolving the EDC or the mixture of EDC and iodoacetamide in a PBS buffer with a concentration of 0.05-0.3 mol/L and a pH value of 5-7, and adding anhydrous ethanol with a volume fraction of 1%-5% to assist the dissolution, wherein the EDC is mixed with the iodoacetamide according to a molar ratio of 1: 1-3: 1.

Preferably, the mixing the modification solution with the phospholipase A₂ for reaction is operated under conditions as follows: a mass ratio of the modification solution to the phospholipase A₂ is 40-80: 50, a reaction temperature is 5-20°C, a stirring speed is 200-500 rpm, and stirring time is 30 minutes-60 minutes.

Preferably, the remaining solution is centrifugally ultrafiltered at 10-30°C and 8000-12000 rpm for 3-10 minutes through an ultrafiltration membrane of 20-50 kDa to obtain the concentrated solution, and then the concentrated solution is diluted by using the PBS with the pH value of 5-7 and the concentration of 0.05-0.3 mol/L.

A second objective of the present application is to provide a specifically chemically-modified phospholipase A₂ obtained by treatment through the method above. After specifically chemically modifying the phospholipase A₂ by the method above, catalytic characteristics of the phospholipase A₂ are improved.

A third objective of the present application is to provide a use of the specifically chemically-modified phospholipase A₂ above in preparation of a high-purity medium chain triglyceride, and to provide a preparation method for the high-purity medium chain triglyceride, in which the specifically chemically-modified phospholipase A₂ above is used to catalyze hydrolysis of coconut oil, which may specifically enrich C8 and C10 fatty acids, and the C8 and C10 fatty acids are esterified with glycerol to obtain the high-purity medium chain triglyceride.

The above objective of the present invention is achieved by the following technical solution.

A preparation method for a medium chain triglyceride includes the following steps:
S1: enzyme-catalyzing hydrolysis of coconut oil by using the specifically chemically-modified phospholipase A₂, and then removing a lower-layer water phase by centrifugation to obtain a mixed hydrolysate containing free fatty acid, monoglyceride, diglyceride and triglyceride;
S2: molecularly distilling the hydrolysate obtained in the step S1 to obtain heavy phase and light phase components respectively; and
S3: mixing the light phase component obtained in the step S2 with glycerol according to a specific ratio, adding a certain amount of molecular sieve, and catalyzing esterification by using a lipase as a catalyst to obtain the medium chain triglyceride.

Preferably, an addition amount of the specifically chemically-modified enzyme in the step S1 is 5-15wt%, and the catalyzing the hydrolysis is operated under conditions as follows: a temperature is 40-80°C, more preferably 60-70°C; and a stirring speed is 500 rpm-700 rpm, and reaction time is 60-180 minutes, more preferably 120 minutes.

Preferably, the centrifugation in the step S1 is operated under conditions as follows: a revolving speed of centrifugation is 4000-8000 rpm, more preferably 7000 rpm, and centrifugation time is 5-30 minutes, more preferably 10 minutes.

Preferably, the molecularly distilling in the step S2 is operated under conditions as follows: a distillation temperature is 150-230°C, more preferably 170°C; and a vacuum degree is 100 pa-1000 pa, a revolving speed of film wiping is 150 rpm-350 rpm, and distillation time is 120-180 minutes.

Preferably, a molar ratio of the light phase component to the glycerol in mixing in the step S3 is 2-4: 1, more preferably 3: 1.

Preferably, an addition amount of the molecular sieve in the step S3 is 0.1-1.5wt‰, more preferably 1.2wt‰. The purpose of adding the molecular sieve in the present application is to moderately remove water molecules produced by the esterification, so that the reaction proceeds in a forward direction, and the occurrence of hydrolysis is reduced.

Preferably, a phosphoric acid may also be added in the esterification in the step S3, and an addition amount of the phosphoric acid is 0.01-0.05 wt%, more preferably 0.02%. The purpose of adding the phosphoric acid in the present application is that the phosphoric acid added in the preparation process plays a role of moderately reversible hydrolysis of a glyceride, a bonding opportunity between short chain fatty acids and the glyceride in the esterification process is improved, and meanwhile, the phosphoric acid serving as a food additive may also play a role of anti-corrosion and metal ion adsorption, thereby reducing inhibition of metal ions on activity of the enzyme in the reaction process, and improving esterification efficiency of the enzyme.

Preferably, the esterification in the step S3 is operated under conditions as follows: the lipase is Novozym 435, and an addition amount of the lipase is 2-20wt%, more preferably 10wt%; and the reaction is operated under conditions as follows: a reaction temperature is 45-85°C, more preferably 65°C; reaction time is 50-100 minutes, more preferably 80 minutes; and a revolving speed of stirring is 200-700 rpm, more preferably 500 rpm.

In the present application, the fatty acids used as raw materials for synthesizing the medium chain triglyceride are obtained by using the modified phospholipase A₂. The advantages of catalyzing the preparation of the medium chain triglyceride by using the modified biological enzyme include high catalytic activity, high product specificity, mild reaction conditions, few by-products, no pollution and unpleasant odors, and environmental protection.

A fourth objective of the present application is to provide a medium chain triglyceride prepared by the method above. In the medium chain triglyceride, fatty acids are mainly C8 and C10 fatty acids, wherein a caprylic-capric triglyceride is a main triglyceride component.

Another objective of the present application is to provide a use of the medium chain triglyceride above as an oil. The oil may be used in the fields of food, nutrition and medicine, such as a rapid energy supplement, a ketogenic diet food, a diluent, an emulsified formulation, and a parenteral nutrition fat emulsion.

Compared with the prior art, the present invention has the beneficial effects as follows.

In the present invention, based on the hydrolysis specificity of the chemically modified phospholipase A₂, the C8 and C10 fatty acids in the natural coconut oil are enriched, and then esterified with the glycerol to synthesize the triglyceride, so as to obtain the high-purity medium chain triglyceride, which contains 95% content of C8 and C10 fatty acids, has metabolic characteristics of medium chain triglyceride, may be rapidly metabolized and absorbed, may serve as a raw material of a fat emulsion used in clinical nutrition to treat and improve malnutrition and malabsorption, and may serve as a main fat component in a ketogenic diet food for rapid ketogenesis.

### DETAILED DESCRIPTION

Technical solutions of the present invention are clearly and completely described hereinafter with reference to embodiments of the present invention. Apparently, the described embodiments are only some but not all of the embodiments of the present invention. Based on the embodiments in the present invention, all other embodiments obtained by those of ordinary skills in the art without going through any creative work should fall within the scope of protection of the present invention.

Unless otherwise specified, all test methods used in the following embodiments are conventional methods; unless otherwise specified, all materials and reagents used may be commercially available reagents and materials; and technological parameters not specified may refer to those of conventional technologies. Lipases used in the embodiment are all purchased from Novozymes A/S (Bagsværd, Denmark).

The present invention aims to replace a traditional chemical method by an enzymatic method, which is a green and safe modification means with little pollution, to prepare a medium chain triglyceride, in which a phospholipase A₂ is specifically chemically-modified to improve catalytic characteristics of the phospholipase A₂, and C8 and C10 fatty acids are specifically enriched, and then esterified with glycerol to obtain the medium chain triglyceride.

### Embodiment 1

A preparation method for a medium chain triglyceride included the following steps.
(1) EDC (1-(3-dimethylaminopropyl)-3-ethylcarbodiimide hydrochloride) was evenly mixed with iodoacetamide according to a molar ratio of 1.6: 1, and then added into a PBS buffer (0.2 mol/L, pH=5.5). The mixture was added with anhydrous ethanol with a volume fraction of 2% to assist the dissolution, and evenly stirred to prepare 0.3 mol/L modification solution. The modification solution was refrigerated at 4°C for later use.
(2) 50 g of the modification solution was mixed with 50 g of phospholipase A₂, and then put into a reactor, wherein a reaction temperature was set at 20°C. When the temperature was constant, the mixture was stirred by a magnetic stirrer (at a revolving speed of 200 rpm), and the reaction was performed under a vacuum condition (at an absolute pressure of 7000 Pa) provided by a water-circulating pump. After reacting for 30 minutes, a specifically modified mixed sample was centrifuged at 20°C and 7000 rpm for 10 minutes, and after removing an upper-layer solvent, a remaining aqueous solution was centrifugally ultrafiltered at 20°C and 10000 rpm for 5 minutes by an ultrafiltration membrane of 30 kDa to obtain a concentrated solution, and then the concentrated solution was diluted with 50 mL of PBS (pH=6.7, 0.1 mol/L) to obtain a specifically chemically-modified phospholipase A₂.
(3) An enzyme solution of the specifically chemically-modified phospholipase A₂ above was physically mixed with coconut oil according to a mass ratio of 1: 10 to react at 70°C, wherein the reaction was performed under a vacuum condition (at an absolute pressure of 7000 Pa) provided by a water-circulating pump and a revolving speed of stirring of 500 rpm. After reacting for 120 minutes, the mixture was centrifuged at 7000 rpm for 10 minutes to remove a lower-layer water phase and obtain a hydrolysate.
(4) The hydrolysate above was molecularly distilled under a distillation temperature of 170°C, a vacuum degree of 100 Pa, a revolving speed of film wiping of 150 rpm, and distillation time of 2 hours to obtain heavy phase and light phase components respectively.
(5) The light phase component above was physically mixed with glycerol according to a molar ratio of 3: 1, and Novozym 435 enzyme was used as a reaction catalyst, which had an addition amount of 10wt%. The mixture was added with 1.2wt‰ molecular sieve and 0.02wt% phosphoric acid to react at a temperature of 65°C and a stirring speed of 500 rpm for 80 minutes. After finishing esterifying, the mixture was centrifuged at 7000 rpm to remove excess lower-layer glycerol and obtain an upper-layer esterification product, which was the medium chain triglyceride.

Determination of fatty acid composition: Shimadzu SH-2560 (100 m×0.2 mm i. d, 0.2 µm) capillary column was adopted, N₂ was used as carrier gas, and a flow rate was 1.5 mL/min. 1 µL of sample was injected according to a split ratio of 40: 1. A temperature of an injection port was set at 250°C, and a temperature of a detector was set at 270°C. Heating procedure: an initial temperature was 80°C, then the temperature was increased to 220°C at 4°C/ min and kept for 5 minutes, and then the temperature was increased to 240°C at 4°C/min and kept for 10 minutes.

Determination of triglyceride composition: Shimadzu SH-65TG (30 m × 0.2 mm i. d, 0.2 µm) capillary column was adopted, H₂ was used as carrier gas, and a flow rate was 1.7 mL/min. 1 µL of sample was injected according to a split ratio of 25: 1. A temperature of an injection port was set at 350°C, and a temperature of a detector was set at 350°C. Heating procedure: an initial temperature was 280°C and kept for 1.5 minutes, then the temperature was increased to 340°C at 10°C/ min and kept for 9.5 minutes, and then the temperature was increased to 350°C at 1°C/min and kept for 12 minutes.

According to the detection and the calculation by the methods above, contents of the C8 and C10 fatty acids in the medium chain triglyceride obtained in this embodiment were 54.2% and 41.4% respectively, wherein contents of triglycerides were 18.2% caprylic triglyceride, 15.6% capric triglyceride and 61.4% caprylic-capric triglyceride respectively.

### Embodiment 2

A preparation method for a medium chain triglyceride included the following steps.

The difference from Embodiment 1 was as follows: in step (5), the light phase component above was physically mixed with glycerol according to a molar ratio of 3: 1, and Novozym 435 enzyme was used as a reaction catalyst, which had an addition amount of 10wt%. The mixture was added with 1.2wt‰ molecular sieve, without a phosphoric acid, to react at a temperature of 65°C and a stirring speed of 500 rpm for 80 minutes. After finishing esterifying, the mixture was centrifuged at 7000 rpm to remove excess lower-layer glycerol and obtain an upper-layer esterification product, which was the medium chain triglyceride.

Other steps and conditions were the same as those of Embodiment 1.

According to the detection and the calculation by the methods in Embodiment 1, contents of the C8 and C10 fatty acids in the medium chain triglyceride obtained in this embodiment were 50.8% and 42.6% respectively, wherein contents of triglycerides were 13.4% caprylic triglyceride, 11.5% capric triglyceride and 58.5% caprylic-capric triglyceride respectively.

### Embodiment 3

A preparation method for a medium chain triglyceride included the following steps.

The difference from Embodiment 1 was as follows: in step (3), an enzyme solution of the specifically chemically-modified phospholipase A₂ was physically mixed with coconut oil according to a mass ratio of 1.5: 10 to react at 70°C, wherein the reaction was performed under a vacuum condition (at an absolute pressure of 7000 Pa) provided by a water-circulating pump and a revolving speed of stirring of 500 rpm. After reacting for 120 minutes, the mixture was centrifuged at 7000 rpm for 10 minutes to remove a lower-layer water phase and obtain a hydrolysate.

Other steps and conditions were the same as those of Embodiment 1.

According to the detection and the calculation by the methods in Embodiment 1, contents of the C8 and C10 fatty acids in the medium chain triglyceride obtained in this embodiment were 47.8% and 37.5% respectively, wherein contents of triglycerides were 9.8% caprylic triglyceride, 15.5% capric triglyceride and 54.3% caprylic-capric triglyceride respectively.

### Embodiment 4

A preparation method for a medium chain triglyceride included the following steps.

The difference from Embodiment 1 was as follows: in step (3), an enzyme solution of the specifically chemically-modified phospholipase A₂ was physically mixed with coconut oil according to a mass ratio of 1:10 to react at 60°C, wherein the reaction was performed under a vacuum condition (at an absolute pressure of 7000 Pa) provided by a water-circulating pump and a revolving speed of stirring of 500 rpm. After reacting for 120 minutes, the mixture was centrifuged at 7000 rpm for 10 minutes to remove a lower-layer water phase and obtain a hydrolysate.

Other steps and conditions were the same as those of Embodiment 1.

According to the detection and the calculation by the methods in Embodiment 1, contents of the C8 and C10 fatty acids in the medium chain triglyceride obtained in this embodiment were 41.7% and 36.3% respectively, wherein contents of triglycerides were 12.5% caprylic triglyceride, 7.5% capric triglyceride and 55.6% caprylic-capric triglyceride respectively.

### Embodiment 5

A preparation method for a medium chain triglyceride included the following steps.

The difference from Embodiment 1 was as follows: in step (1), EDC (1-(3-dimethylaminopropyl)-3-ethylcarbodiimide hydrochloride) was evenly mixed with iodoacetamide according to a molar ratio of 2.5: 1, and then added into a PBS buffer (0.2 mol/L, pH=5.5). The mixture was added with anhydrous ethanol with a volume fraction of 2% to assist the dissolution, and evenly stirred to prepare 0.3 mol/L modification solution. The modification solution was refrigerated at 4°C for later use.

Other steps and conditions were the same as those of Embodiment 1.

According to the detection and the calculation by the methods in Embodiment 1, contents of the C8 and C10 fatty acids in the medium chain triglyceride obtained in this embodiment were 39.4% and 29.6% respectively, wherein contents of triglycerides were 11.8% caprylic triglyceride, 10.6% capric triglyceride and 41.8% caprylic-capric triglyceride respectively.

### Embodiment 6

A preparation method for a medium chain triglyceride included the following steps.

The difference from Embodiment 1 was as follows: in step (5), the light phase component above was physically mixed with glycerol according to a molar ratio of 2: 1, and Novozym 435 enzyme was used as a reaction catalyst, which had an addition amount of 2wt%. The mixture was added with 1.2wt‰ molecular sieve and 0.02wt% phosphoric acid to react at a temperature of 65°C and a stirring speed of 500 rpm for 80 minutes. After finishing esterifying, the mixture was centrifuged at 7000 rpm to remove excess lower-layer glycerol and obtain an upper-layer esterification product, which was the medium chain triglyceride.

Other steps and conditions were the same as those of Embodiment 1.

According to the detection and the calculation by the methods in Embodiment 1, contents of the C8 and C10 fatty acids in the medium chain triglyceride obtained in this embodiment were 35.6% and 25.1% respectively, wherein contents of triglycerides were 9.8% caprylic triglyceride, 12.5% capric triglyceride and 36.4% caprylic-capric triglyceride respectively.

### Embodiment 7

A preparation method for a medium chain triglyceride included the following steps.

The difference from Embodiment 1 was as follows: in step (1), EDC (1-(3-dimethylaminopropyl)-3-ethylcarbodiimide hydrochloride) was added into a PBS buffer (0.2 mol/L, pH=5.5). The mixture was added with anhydrous ethanol with a volume fraction of 2% to assist the dissolution, and evenly stirred to prepare 0.3 mol/L modification solution. The modification solution was refrigerated at 4°C for later use.

Other steps and conditions were the same as those of Embodiment 1.

According to the detection and the calculation by the methods in Embodiment 1, contents of the C8 and C10 fatty acids in the medium chain triglyceride obtained in this embodiment were 33.7% and 27.4% respectively, wherein contents of triglycerides were 9.5% caprylic triglyceride, 13.4% capric triglyceride and 38.5% caprylic-capric triglyceride respectively.

### Comparative Example 1

A preparation method for a medium chain triglyceride included the following steps.

The difference from Embodiment 1 was as follows: there was not the operation of specifically modifying the phospholipase A₂ by using the modification solution in the step (2). Other steps and conditions were the same as those of Embodiment 1.

According to the detection and the calculation by the methods in Embodiment 1, contents of the C8 and C10 fatty acids in the medium chain triglyceride obtained in this embodiment were 14.5% and 14.1% respectively, wherein contents of triglycerides were 5.6% caprylic triglyceride, 6.7% capric triglyceride and 17.8% caprylic-capric triglyceride respectively.

### Comparative Example 2

A preparation method for a medium chain triglyceride included the following steps.

The difference from Embodiment 1 was as follows: there was not the operation of specifically modifying the phospholipase A₂ by using the modification solution in the step (2). In step (3), an enzyme solution of an unmodified phospholipase A₂ with the same concentration was physically mixed with coconut oil according to a mass ratio of 1.5: 10 to react at 70°C, wherein the reaction was performed under a vacuum condition (at an absolute pressure of 7000 Pa) provided by a water-circulating pump and a revolving speed of stirring of 500 rpm. After reacting for 120 minutes, the mixture was centrifuged at 7000 rpm to remove a lower-layer water phase and obtain a hydrolysate.

Other steps and conditions were the same as those of Embodiment 1.

According to the detection and the calculation by the methods in Embodiment 1, contents of the C8 and C10 fatty acids in the medium chain triglyceride obtained in this embodiment were 13.6% and 17.5% respectively, wherein contents of triglycerides were 7.5% caprylic triglyceride, 4.3% capric triglyceride and 15.5% caprylic-capric triglyceride respectively.

### Comparative Example 3

A preparation method for a medium chain triglyceride included the following steps.

The difference from Embodiment 1 was as follows: in step (1), PEG (polyethylene glycol) was evenly mixed with iodoacetamide according to a molar ratio of 1.6: 1, and then added into a PBS buffer (0.2 mol/L, pH=5.5). The mixture was added with anhydrous ethanol with a volume fraction of 2% to assist the dissolution, and evenly stirred to prepare 0.3 mol/L modification solution. The modification solution was refrigerated at 4°C for later use.

Other steps and conditions were the same as those of Embodiment 1.

According to the detection and the calculation by the methods in Embodiment 1, contents of the C8 and C10 fatty acids in the medium chain triglyceride obtained in this embodiment were 12.7% and 12.6% respectively, wherein contents of triglycerides were 6.8% caprylic triglyceride, 7.5% capric triglyceride and 16.7% caprylic-capric triglyceride respectively.

### Comparative Example 4

A preparation method for a medium chain triglyceride included the following steps.

The difference from Embodiment 1 was as follows: there was not the operation of specifically modifying the phospholipase A₂ by using the modification solution in the step (2). In step (4), the hydrolysate above was molecularly distilled under a distillation temperature of 190°C, a vacuum degree of 100 Pa, a revolving speed of film wiping of 150 rpm, and distillation time of 2 hours to obtain heavy phase and light phase components respectively.

Other steps and conditions were the same as those of Embodiment 1.

According to the detection and the calculation by the methods in Embodiment 1, contents of the C8 and C10 fatty acids in the medium chain triglyceride obtained in this embodiment were 16.8% and 16.2% respectively, wherein contents of triglycerides were 8.5% caprylic triglyceride, 3.6% capric triglyceride and 18.6% caprylic-capric triglyceride respectively.

### Comparative Example 5

A preparation method for a medium chain triglyceride included the following steps.

The difference from Embodiment 1 was as follows: in step (1), iodoacetamide was added into a PBS buffer (0.2 mol/L, pH=5.5). The mixture was added with anhydrous ethanol with a volume fraction of 2% to assist the dissolution, and evenly stirred to prepare 0.3 mol/L modification solution. The modification solution was refrigerated at 4°C for later use.

Other steps and conditions were the same as those of Embodiment 1.

According to the detection and the calculation by the methods in Embodiment 1, contents of the C8 and C10 fatty acids in the medium chain triglyceride obtained in this embodiment were 20.3% and 16.4% respectively, wherein contents of triglycerides were 8.5% caprylic triglyceride, 4.3% capric triglyceride and 21.7% caprylic-capric triglyceride respectively.

In order to further illustrate effects of the solutions of the present application, intermediate products and final products prepared by the solutions of the embodiments and the comparative examples were subjected to component analysis.

Table 1 shows fatty acid composition data of light phase components of the intermediate products in the embodiments and the comparative examples after molecular distillation.

Table 2 shows fatty acid composition data of the final products in the embodiments and the comparative examples.

Table 3 shows triglyceride composition data of the final products in the embodiments and the comparative examples.

**Table 1**

| Sample (%) | C8:0 | C10:0 | C12:0 | C14:0 | C16:0 | C18:0 | C18:1 | C18:2 | Others |
|---|---|---|---|---|---|---|---|---|---|
| Embodiment 1 | 48.6 | 37.2 | 6.8 | 2.7 | 0.7 | 0.9 | 1.5 | 1.4 | 0.2 |
| Embodiment 2 | 48.4 | 37.3 | 7.0 | 3.1 | 0.5 | 0.8 | 1.5 | 1.1 | 0.3 |
| Embodiment 3 | 35.9 | 29.8 | 9.5 | 11.8 | 2.3 | 2.6 | 1.3 | 5.7 | 1.1 |
| Embodiment 4 | 38.4 | 30.3 | 13.8 | 6.8 | 1.5 | 3.4 | 0.7 | 3.8 | 1.3 |
| Embodiment 5 | 36.5 | 28.4 | 17.3 | 6.9 | 3.4 | 1.4 | 2.2 | 3.5 | 0.4 |
| Embodiment 6 | 31.1 | 22.5 | 29.5 | 10.7 | 0.4 | 1.6 | 1.1 | 2.2 | 0.9 |
| Embodiment 7 | 30.7 | 26.5 | 30.9 | 0.3 | 1.8 | 3.7 | 2.1 | 2.8 | 1.2 |
| Comparative Example 1 | 11.7 | 10.2 | 42.3 | 9.3 | 12.5 | 8.2 | 1.7 | 2.3 | 1.8 |
| Comparative Example 2 | 9.1 | 13.2 | 40.5 | 11.9 | 7.6 | 7.9 | 5.1 | 2.5 | 2.2 |
| Comparative Example 3 | 7.2 | 7.6 | 41.4 | 15.2 | 9.5 | 6.7 | 6.5 | 3.6 | 2.3 |
| Comparative Example 4 | 11.8 | 9.7 | 39.5 | 17.2 | 6.4 | 8.5 | 3.7 | 1.7 | 1.5 |
| Comparative Example 5 | 16.2 | 12.7 | 36.3 | 9.8 | 8.3 | 7.6 | 4.2 | 2.5 | 2.4 |

**[Table 2]**

| Sample (%) | C8:0 | C10:0 | C12:0 | C14:0 | C16:0 | C18:0 | C18:1 | C18:2 | Others |
|---|---|---|---|---|---|---|---|---|---|
| Coconut oil | 8.7 | 7.8 | 43.2 | 16.2 | 9.4 | 2.6 | 6.7 | 1.6 | 3.8 |
| Embodiment 1 | 54.2 | 41.4 | 1.8 | 1.1 | 0.6 | 0.2 | 0.2 | 0.2 | 0.3 |
| Embodiment 2 | 50.8 | 42.6 | 2.1 | 1.5 | 0.4 | 0.3 | 0.5 | 0.5 | 1.3 |
| Embodiment 3 | 47.8 | 37.5 | 4.5 | 2.6 | 1.7 | 1.1 | 2.3 | 0.7 | 1.8 |
| Embodiment 4 | 41.7 | 36.3 | 10.7 | 2.3 | 1.4 | 1.3 | 2.9 | 1.5 | 1.9 |
| Embodiment 5 | 39.4 | 29.6 | 21.7 | 2.3 | 1.6 | 0.5 | 1.6 | 1.1 | 2.2 |
| Embodiment 6 | 35.6 | 25.1 | 25.4 | 3.7 | 2.5 | 0.9 | 2.8 | 1.7 | 2.3 |
| Embodiment 7 | 33.7 | 27.4 | 25.9 | 3.5 | 1.8 | 1.2 | 3.2 | 1.6 | 1.7 |
| Comparative Example 1 | 14.5 | 14.1 | 37.8 | 13.6 | 7.9 | 1.8 | 4.7 | 1.5 | 4.1 |
| Comparative Example 2 | 13.6 | 17.5 | 35.8 | 14.2 | 6.7 | 2.2 | 4.1 | 1.4 | 4.5 |
| Comparative Example 3 | 12.7 | 12.6 | 39.5 | 12.8 | 8.5 | 2.3 | 5.6 | 1.3 | 4.7 |
| Comparative Example 4 | 16.8 | 16.2 | 36.4 | 12.7 | 7.3 | 1.5 | 4.6 | 0.7 | 3.8 |
| Comparative Example 5 | 20.3 | 16.4 | 34.7 | 8.5 | 6.5 | 2.2 | 5.8 | 1.8 | 3.8 |

**Table 3**

| Contents of triglycerides (%) | Caprylic triglyceride | Capric triglyceride | Caprylic-capric triglyceride |
|---|---|---|---|
| Embodiment 1 | 18.2 | 15.6 | 61.4 |
| Embodiment 2 | 13.4 | 11.5 | 58.5 |
| Embodiment 3 | 9.8 | 15.5 | 54.3 |
| Embodiment 4 | 12.5 | 7.5 | 55.6 |
| Embodiment 5 | 11.8 | 10.6 | 41.8 |
| Embodiment 6 | 9.8 | 12.5 | 36.4 |
| Embodiment 7 | 9.5 | 13.4 | 38.5 |
| Comparative Example 1 | 5.6 | 6.7 | 17.8 |
| Comparative Example 2 | 7.5 | 4.3 | 15.5 |
| Comparative Example 3 | 6.8 | 7.5 | 16.7 |
| Comparative Example 4 | 8.5 | 3.6 | 18.6 |
| Comparative Example 5 | 8.5 | 4.3 | 21.7 |

In conclusion, the phospholipase A₂ is specifically chemically-modified through the prepared modification solution to obtain the modified phospholipase A₂, which is employed as a catalyst to selectively catalyze the hydrolysis of the coconut oil, and the light phase component obtained after molecular distillation of the hydrolysate is used as a raw material to synthesize the medium chain triglyceride. The results indicate that, compared with the phospholipase A₂, the specifically chemically-modified phospholipase A₂ shows improved hydrolysis selectivity for the C8 and C10 fatty acids in the coconut oil, so that the light phase component having the high content of C8 and C10 fatty acids is obtained after molecular distillation of the hydrolysate, and by means of subsequent esterification with the glycerol, the medium chain triglyceride in which the content of C8 and C10 reaches 95% can be obtained, and used in the fields of food, nutrition, medicine, etc. Among all the embodiments, the final medium chain triglyceride obtained by mixing the specifically chemically-modified phospholipase A₂ with the coconut oil according to the mass ratio of 1:10 in Embodiment 1 is the best.

The above embodiments of the present invention are only examples for clearly explaining the technical solutions of the present invention, and are not intended to limit all the specific implementations of the present invention. Any modifications, equivalent substitutions, improvements, etc. made within the spirit and principle of the claims of the present invention should all fall within the scope of protection of the claims of the present invention.

## Claims

1. A method for improving a catalytic activity of a specifically chemically-modified phospholipase A₂, wherein the method comprises the following steps: mixing a modification solution with a phospholipase A₂ for reaction, removing an upper-layer solvent by centrifugation after the reaction, centrifugally ultrafiltering a remaining solution to obtain a concentrated solution, and diluting the concentrated solution to obtain the specifically chemically-modified phospholipase A₂, wherein the modification solution is prepared from 1-(3-dimethylaminopropyl)-3-ethylcarbodiimide hydrochloride or a mixture of 1-(3-dimethylaminopropyl)-3-ethylcarbodiimide hydrochloride and iodoacetamide.

2. The method according to claim 1, wherein the modification solution is a modification solution with a concentration of 0.1-0.5 mol/L, which is prepared by dissolving the 1-(3-dimethylaminopropyl)-3-ethylcarbodiimide hydrochloride or the mixture of 1-(3-dimethylaminopropyl)-3-ethylcarbodiimide hydrochloride and iodoacetamide mixed according to a molar ratio of 1: 1-3: 1 in a PBS buffer with a concentration of 0.05-0.3 mol/L and a pH value of 5-7, and adding anhydrous ethanol with a volume fraction of 1%-5% to assist the dissolution.

3. The method according to claim 1, wherein the mixing for reaction is operated under conditions as follows: a mass ratio of the modification solution to the phospholipase A₂ is 40-80: 50, a reaction temperature is 5-20°C, a stirring speed is 200-500 rpm, and stirring time is 30 minutes-60 minutes.

4. The method according to claim 1, wherein the remaining solution is centrifugally ultrafiltered at 10-30°C and 8000-12000 rpm for 3-10 minutes through an ultrafiltration membrane of 20-50 kDa to obtain the concentrated solution, and then the concentrated solution is diluted by using the PBS with the pH value of 5-7 and the concentration of 0.05-0.3 mol/L.

5. A specifically chemically-modified phospholipase A₂ obtained by treatment through the method according to any one of claims 1 to 4.

6. A preparation method for a medium chain triglyceride, comprising the following steps:
S1: enzyme-catalyzing hydrolysis of coconut oil by using the specifically chemically-modified phospholipase A₂ according to claim 5, and removing a lower-layer water phase by centrifugation to obtain a hydrolysate;
S2: molecularly distilling the hydrolysate obtained in the step S1 to obtain heavy phase and light phase components respectively; and
S3: mixing the light phase component obtained in the step S2 with glycerol, adding a molecular sieve, and catalyzing esterification by using a lipase to obtain the medium chain triglyceride.

7. The preparation method according to claim 6, wherein an addition amount of the enzyme in the step S1 is 5-15wt%, and the catalyzing the hydrolysis is operated under conditions as follows: a temperature is 40-80°C, a stirring speed is 500 rpm-700 rpm, and reaction time is 60-180 minutes, and the centrifugation is operated under conditions as follows: a revolving speed is 4000-8000 rpm, and centrifugation time is 5-30 minutes.

8. The preparation method according to claim 6, wherein the molecularly distilling in the step S2 is operated under conditions as follows: a distillation temperature is 150-230°C; and a vacuum degree is 100 pa-1000 pa, a revolving speed of film wiping is 150 rpm-350 rpm, and distillation time is 120-180 minutes.

9. The preparation method according to claim 6, wherein a molar ratio of the light phase component to the glycerol in mixing in the step S3 is 2-4: 1; an addition amount of the molecular sieve is 0.1-1.5wt‰; a phosphoric acid is capable of being added in the esterification, and an addition amount of the phosphoric acid is 0.01-0.05 wt%; the lipase is Novozym 435, and an addition amount of the lipase is 2-20wt%; and the reaction is operated under conditions as follows: a reaction temperature is 45-85°C, reaction time is 50-100 minutes, and a revolving speed of stirring is 200-700 rpm.

10. A medium chain triglyceride prepared by the method according to any one of claims 6 to 9.
